(19) Europäisches Patentamt
European Patent Office
Office européen des brevets

(11) EP 0 906 261 B1

(12) **EUROPÄISCHE PATENTSCHRIFT**

(45) Veröffentlichungstag und Bekanntmachung des Hinweises auf die Patenterteilung:
**09.05.2001 Patentblatt 2001/19**

(21) Anmeldenummer: **97925959.5**

(22) Anmeldetag: **28.05.1997**

(51) Int Cl.[7]: **C07C 45/50**, C07F 9/6568, B01J 31/24

(86) Internationale Anmeldenummer:
**PCT/EP97/02757**

(87) Internationale Veröffentlichungsnummer:
**WO 97/46507 (11.12.1997 Gazette 1997/53)**

(54) **VERFAHREN ZUR HYDROFORMYLIERUNG UND DAFÜR GEEIGNETE KATALYSATOREN, DIE PHOSPHORVERBINDUNGEN ALS LIGANDEN ENTHALTEN**

HYDROFORMYLATION PROCESS AND CATALYSTS SUITABLE THEREFOR CONTAINING PHOSPHORUS COMPOUNDS AS LIGANDS

PROCEDE D'HYDROFORMYLATION ET CATALYSEURS APPROPRIES A CET EFFET, CONTENANT DES COMPOSES PHOSPHORE COMME LIGANDS

(84) Benannte Vertragsstaaten:
**BE DE ES FR GB IT NL**

(30) Priorität: **31.05.1996 DE 19621967**

(43) Veröffentlichungstag der Anmeldung:
**07.04.1999 Patentblatt 1999/14**

(73) Patentinhaber: **BASF AKTIENGESELLSCHAFT**
**67056 Ludwigshafen (DE)**

(72) Erfinder:
- **BREIT, Bernhard**
  **D-35041 Marburg (DE)**
- **PACIELLO, Rocco**
  **D-67098 Bad Dürkheim (DE)**
- **GEISSLER, Bernhard**
  **D-67281 Kirchheim (DE)**
- **RÖPER, Michael**
  **D-67157 Wachenheim (DE)**

(56) Entgegenhaltungen:
**EP-A- 0 646 588** **DE-B- 1 668 622**

- **CHEM. COMMUN. (CAMBRIDGE) (CHCOFS,13597345);96; (17); PP.2071-2072, PHILIPPS-UNIV. MARBURG;FACHBEREICH CHEMIE; MARBURG; D-35043; GERMANY (DE), XP002039594 BREIT B: "Highly regioselective hydroformylation under mild conditions with new classes of.pi.-acceptor ligands"**
- **TETRAHEDRON LETT. (TELEAY,00404039);95; VOL.36 (22); PP.3839-42, UNIV. REGENSBURG;INST. ORG. CHEM.; REGENSBURG; D-93040; GERMANY (DE), XP002039595 MAERKL G ET AL: "3.lambda.3,3'.lambda.3-Diphosphinins. 3.lambda.2.sigma.2,3''.lambda.2.sigma.2-Di phospha-o,m,p-terphenyls. 3.lambda.3.sigma.2,3'''.lambda.3.sigma.2-D iphospha-o,p-quaterphenyls"**

EP 0 906 261 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft ein Verfahren zur Hydroformylierung von Alkenen mit Komplexen von Metallen der VIII. Nebengruppe des Periodischen Systems der Elemente und mit Phosphaaromaten mit 5 bis 100 C-Atomen als Liganden, sowie diese neuen Komplexkatalysatoren.

Jährlich werden weltweit etwa 7 mio Tonnen Produkte mit Hilfe der Hydroformylierung von Olefinen hergestellt (Weissermel, Arpe Industrielle Organische Chemie 4. Auflage 1994 VCH Weinheim). Es besteht deshalb ein großes wirtschaftliches Interesse, die Reaktion mit maximaler Selektivität und Aktivität zu betreiben.

Rhodiumhaltige phosphinmodifizierte Katalysatoren haben insbesondere für die niedersiedenden Olefine eine überragende Bedeutung gewonnen (z.B. Beller et al Journal of Molecular Catalysis A: 104 (1995)17-85). Obwohl α-Olefine sehr gut mit rhodiumhaltigen phosphinmodifizierten Katalysatoren hydroformylierbar sind (vgl. J. Falbe, Ed.: New Syntheses With Carbon Monoxide, Springer, Berlin 1980, S. 55 ff) ist dieses Katalysatorsystem für interne und interne, verzweigte Olefine sowie für Olefine mit mehr als 7 Kohlenstoffatomen wenig geeignet (vgl. Falbe, S. 95 ff). So werden interne Kohlenstoff-Kohlenstoff-Doppelbindungen nur sehr langsam in Gegenwart eines derartigen Katalysators hydroformyliert.

Rhodiumhaltige phosphitmodifizierte Katalysatoren werden neuerdings für die Hydroformylierung von niedersiedenden Olefinen vorgeschlagen (s. Beller et al Journal of Molecular Catalysis a.a.O.) Sowohl α-Olefine als auch kurzkettige interne Olefine, wie 2-Buten oder 3-Pentensäuremethylester, können sehr gut mit rhodiumhaltigen Chelatphosphitmodifizierten Katalysatoren hydroformyliert werden. Besonders hohe Linearitäten werden mit Chelatliganden mit einem Bißwinkel von etwa 120° erzielt. Allerdings ist dieses System für langkettig-interne Olefine (mit mehr als 7 Kohlenstoffatomen) und interne, verzweigte Olefine wenig geeignet. Für diese Olefine haben sich einzähnige, sterisch gehinderte Monophosphite bewährt.

Allerdings weisen Phosphite generell den Nachteil der Hydrolyseempfindlichkeit und der Tendenz zu Abbaureaktionen auf, wodurch deren technischer Einsatz behindert wird.

DE-AS 1 668 622 betrifft ein Verfahren zur Hydroformylierung von Olefinen mittels Katalysatoren aus Komplexen von Übergangsmetallen der VIII. Nebengruppe mit Liganden, die eine P=C-Doppelbindung enthalten, insbesondere mit offenkettigen Liganden, offenbart jedoch nicht den Einsatz von Phosphaaromaten als Liganden.

EP-A 646 588 betrifft die Verwendung von cyclischen Phosphor(III)verbindungen, insbesondere von Phospholanen, Phospholinen und Phospholen, als zweizähnige Liganden für Komplexverbindungen mit zahlreichen Metallatomen, darunter Rhodium.

Der vorliegenden Erfindung lag daher die Aufgabe zugrunde, neue, gegen Hydrolyse und Abbaureaktionen unempfindliche Liganden, die als komplex gebundene Cokatalysatoren für die durch Übergangsmetalle der VIII. Nebengruppe, insbesondere Rhodium, katalysierte Hydroformylierung von Olefinen besonders geeignet sind, sowie ein Verfahren zur Herstellung von Aldehyden aus α-Olefinen, aus internen und/oder verzweigten Olefinen oder aus höhersiedenden Olefinen (mit mehr als 7 Kohlenstoffatomen) zu finden.

Diese Aufgabe wurde gelöst mit einem Verfahren zur Herstellung von Aldehyden durch Hydroformylierung von Olefinen mit $CO/H_2$ in Gegenwart von Phosphorverbindungen als Liganden enthaltenden Komplexen von Übergangsmetallen der Nebengruppe VIII des Periodischen Systems der Elemente bei Temperaturen von 20 bis 200°C, vorzugsweise 20 bis 180°C und Drücken bis 700 bar, vorzugsweise bis 300 bar, das dadurch gekennzeichnet ist, daß man als Liganden Phosphaaromaten mit 5 bis 100 C-Atomen verwendet.

Dabei sind die katalytisch aktiven Komplexe Carbonylkomplexe, die sich durch zusätzliche Anlagerung von Kohlenmonoxid bilden.

Als carbonylgruppenfreie Komplexe von Metallen der VIII. Nebengruppe mit Phosphabenzol als Ligand sind schon Tetrakis(phosphabenzol)nickel aus Angew. Chem., 1992, 104, S. 1388 ff und Chlorhaltige Komplexe u.a. Tris (phosphabenzol)rhodiumchlorid und Bis(phosphabenzol)rhodiumchloriddimer aus J. Heterocyclic Chem., 1972, Band 9, S. 1457 ff bekannt, ohne daß eine Anwendung in der Katalysechemie, insbesondere in der Hydroformylierung beschrieben wurde.

Obwohl viele phosphorhaltige Liganden für die Rhodium-Niederdruck-Hydroformylierung bekannt sind (u.a. Arylphosphine, Alkylphosphine, Chelatphosphine, Monophosphite, Chelatphosphite, Phosphole), wurde der Einsatz von Phosphabenzolen bisher nicht erwogen. Bei unseren Versuchen fanden wir nun, daß diese Liganden als Cokatalysatoren nicht nur für die Hydroformylierung von α-Olefinen hochaktiv und damit hervorragend geeignet sind, sondern auch für die Hydroformylierung interner, verzweigter Olefine, wie z.B. Octen-N, ein Isooctengemisch mit Verzweigungsgrad 1,3, das durch die Dimerisierung von Buten nach dem IFP-Dimersol-Prozeß (s. Weissermel, Arpe: Industrielle Organische Chemie, S. 93, 4. Auflage, VCH Verlagsgesellschaft, Weinheim 1994) enthalten wird, aktiv sind. Der Verzweigungsgrad ist als das arithmetische Mittel der Anzahl der Seitenketten aller Molekülspezies einer Olefinmischung multipliziert mit deren jeweiligen Gehalt in der Olefinmischung in Gew.-%, dividiert durch 100, definiert.

Als erfindungsgemäß zu verwendende Liganden kommen Phosphaaromaten mit mindestens 5, z.B. 7 bis 100 C-Atomen in Betracht. Insbesondere kommen gegebenenfalls substituierte Phosphabenzole der Formel I in Be-

tracht

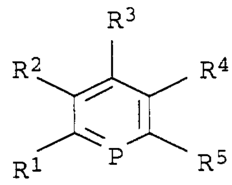

I,

in der $R^1$ - $R^5$ unabhängig voneinander für Wasserstoff, Alkyl-Aralkyl- oder Arylreste oder heterocyclische Reste mit jeweils 1 - 12 C-Atomen steht, ferner für die Reste $-COO^{\ominus}M^{\oplus}$, $-SO_3^{\ominus}M^{\oplus}$ ,

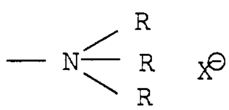

-OR, $-NR_2$, -COOR, -SR, $-(CHRCH_2O)_xR$ und $(CH_2CH_2NR)_x$, wobei R gleich oder verschiedene Reste ausgewählt aus Wasserstoff oder aliphatischen oder aromatischen Resten mit 1 bis 18 C-Atomen, $M^{\oplus}$ ein Kation, z.B. Alkali oder Ammonium und $X^{\ominus}$ ein Anion, z.B. Sulfat und x die Zahlen 1 bis 120 bedeuten und wobei die Reste $R^1$ - $R^5$ zu annellierten Ringen verbunden sein können.

Mindestens einer der Substituenten $R^1$ bis $R^5$, kann eine zusätzliche Phosphin- oder Phosphitgruppierung aufweisen und wird dadurch zu einem zwei- oder mehrzähnigen Ligand.

Die erfindungsgemäß bevorzugt zu verwendenden 2- oder mehrzähnigen Liganden weisen jedoch 2 oder mehr Phosphabenzoleinheiten auf, die durch ein Brückenglied verbunden sind. Verbindungen dieser Art sind solche der Formel II

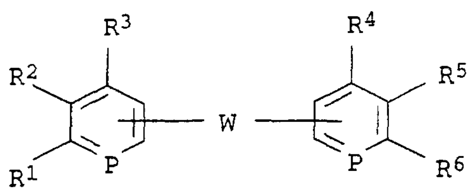

II,

in der $R_1$-$R_6$ die oben für $R_1$-$R_5$ angegebenen Bedeutungen haben und W eine Brücke in Form einer kovalenten Bindung oder einer Kette aus 1 bis 10, vorzugsweise bis 6 Atomen bedeutet, die Bestandteil einer cyclischen oder aromatischen Verbindung sein kann und wobei die Phosphaaromaten in ortho- oder meta-Stellung zum Phosphor verknüpft sind, wobei die nicht für die Brücke verwendeten ortho- oder meta-Positionen durch Reste wie $R^1$ bis $R^5$ der Formel I substituiert sind, und wobei einer oder mehrere der Substituenten $R^1$ bis $R^6$ ein über eine weitere Brücke gebundenes Phosphabenzol der Formel I bedeuten kann.

Solche atomare Glieder (Atome) der Brücke W können z.B. Methylengruppen der Formel

$$-CR^7R^8,$$

in der $R^7$ und $R^8$ gleich oder verschieden sind und für Wasserstoff, eine $C_1$- bis $C_9$-Alkylgruppe, insbesondere die Methylgruppe, eine $C_6$- bis $C_{12}$-Arylgruppe, insbesondere die Phenylgruppe, oder eine $C_7$- bis $C_{15}$-Aralkylgruppe, insbesondere die Benzylgruppe, stehen, die Oxagruppe -O-, die Aminogruppe

$$-NR^9 ,$$

in der $R^9$ für eine $C_1$- bis $C_4$-Alkylgruppe, insbesondere die Methylgruppe, eine $C_6$- bis $C_{12}$-Arylgruppe, insbesondere die Phenylgruppe, oder eine $C_7$- bis $C_{15}$-Aralkylgruppe, insbesondere die Benzylgruppe, steht oder eine Silylgruppe

der Formel

$$-SiR^{10}R^{11} ,$$

in der die Reste $R^{10}$ und $R^{11}$ gleich oder verschieden sind und für eine $C_1$- bis $C_4$-Alkylgruppe, insbesondere die Methylgruppe, eine $C_6$- bis $C_{12}$-Arylgruppe, insbesondere die Phenylgruppe, eine $C_7$-bis $C_{15}$-Aralkylgruppe, insbesondere die Benzylgruppe, stehen, sein.

Als cyclische oder aromatische Verbindungen, die zusammen mit der kovalenten Bindung oder den vorgenannten atomaren Brückengliedern Bestandteil der Brücke W sein können, seien die $C_5$ - bis $C_{10}$-Cycloalkyldiyl-Gruppen, insbesondere die Cyclopentandiyl-, die Cyclohexyldiyl- oder die Decalindiyl-Gruppe, $C_6$- bis $C_{12}$-Aryldiylgruppen, wie die Phenylen-, Naphthylen- oder 1,1'-Diphenyl-diyl-Gruppe, die Diphenylether-diyl-Gruppe

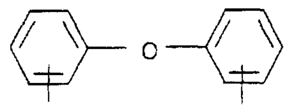 ,

die Diphenylmethan-diyl-Gruppe

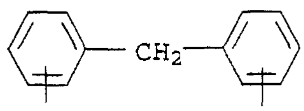 ,

die Diphenylethan-diyl-Gruppe

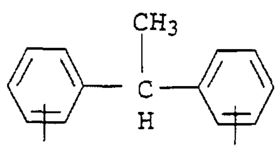

die Diphenylpropan-diyl-Gruppe

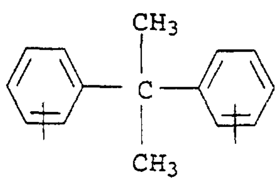

oder auch die Ferrocen-diyl-Gruppe genannt.

Beispielhaft seien folgende Phosphabenzole genannt:

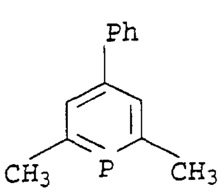 , 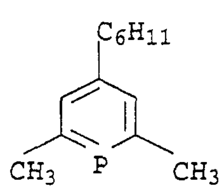 , 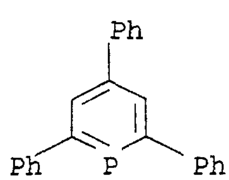 , 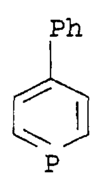 ,

Ligand 1

Ligand 3

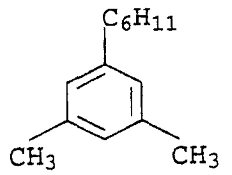 , 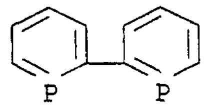 , 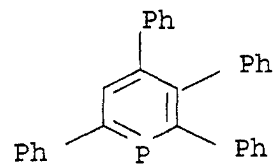

Ligand 2

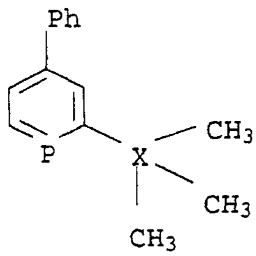 , 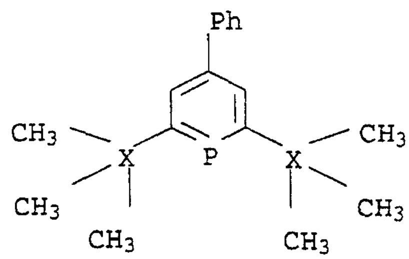

Ph = Phenyl X = C, Si X = C, Si

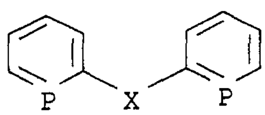 , 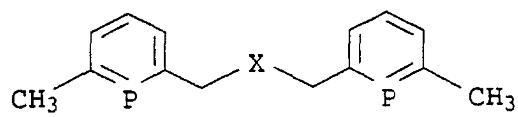

X = $CH_2$, $SiMe_2$, O X = $CH_2$, $(CH_2)_2$, $SiMe_2$, O ,

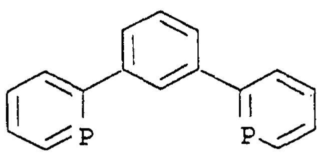 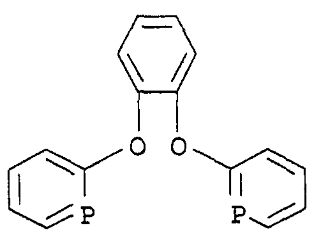 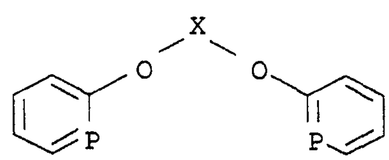

X=$CH_2$, $(CH_2)_2$, $SiMe_2$

Besonders vorteilhaft erweisen sich im erfindungsgemäßen Verfahren als zweizähnige Liganden die Phosphabenzole der allgemeinen Formel IIa

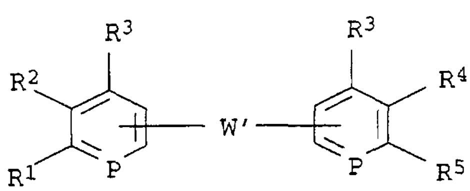 IIa,

in der die Reste $R^1$ bis $R^6$ gleich oder verschieden sind und die vorstehend für die Reste $R^1$ bis $R^5$ genannte Bedeutung haben und in der W' eine 2,2'-, 4,4'- oder 3,3'-Diphenyldiyl-Gruppe, eine 2,2'-, 4,4' - oder 3,3'-Diphenylether-diylgruppe, eine 2,2'-, 4,4'- oder 3,3'-Diphenylmethan-diyl-, eine 2,2'-, 4,4'- oder 3,3'-Diphenylethan-diyl-gruppe oder eine 2,2'-, 4,4'- oder 3,3'-Diphenylpropan-diyl-Gruppe ist, die mit den beiden Phosphabenzolgruppen in ortho- oder meta-Stellung zu deren Phosphoratom jeweils über eine kovalente Bindung, eine Oxa-Gruppe und/oder eine $C_1$- bis $C_4$-Alkylgruppe, insbesondere eine Methylengruppe verknüpft ist, worin die Diphenyl-, Diphenylether- oder $C_1$- bis $C_3$-Diphenylalkan-Gruppierung unsubstituiert oder mit 1 bis 4 gleichen oder verschiedenen $C_1$- bis $C_4$-Alkylgruppen substituiert ist, und wobei die nicht für die Brücke verwendeten ortho- oder meta-Positionen durch Reste wie $R^1$ bis $R^5$ der Formel I substituiert sind. Es versteht sich aus dem Vorstehenden, daß die betreffenden Diphenylderivat-Gruppierungen mit den beiden Phosphabenzolgruppen asymmetrisch, d.h. über zwei unterschiedliche Verbindungsglieder, verknüpft sein können.

Ein besonders vorteilhafter Ligand aus der Gruppe der Phosphabenzole der allgemeinen Formel IIa ist das Phosphabenzol IV, in den Beipielen auch als Ligand 5 bezeichnet

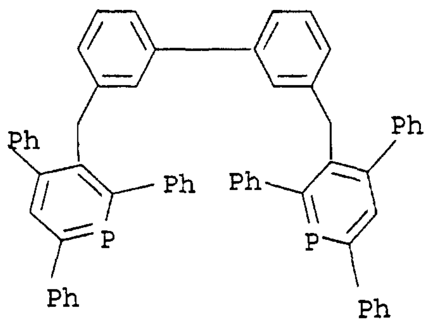

IV,

in der Ph die Abkürzung für eine Phenylgruppe ist.

Die Liganden der Formel IIa, insbesondere der Ligand der Formel IV zeichnen sich dadurch aus, daß sie praktisch nur endständige Doppelbindungen hydroformylieren und eine höhere Selektivität für die Bildung von linearen Aldehyden, d.h. eine höhere n-Selektivität haben.

Das Prinzip der Herstellung der Phosphabenzole ist bekannt. Generelles zur Synthese von Phosphabenzolen findet sich in Houben-Weyl, Phosphororganische Verbindungen, Hrsg. M. Regitz, Band 2, S. 72 ff. Noch nicht im einzelnen beschriebene Verbindungen werden analog erhalten.

Die wirksamen neuen Katalysatoren sind solche der Formel III $M(L)_n(CO)_m$, in der M mindestens ein Zentralatom eines Elements der VIII. Nebengruppe des periodischen Systems der Elemente, L mindestens ein Ligand mit einer zur Komplexbildung befähigten Gruppierung beispielsweise der Formeln I, II, IIa oder der Ligand der Formel IV, n und m jeweils mindestens 1 ist und wobei n und m pro Äquivalent M z.B. jeweils die Zahlen 1 bis 3 und die Summe von n+m 2 bis 5 bedeuten und wobei noch weitere Reste wie Hydrido oder Alkyl- oder Acylreste als Liganden enthalten sein können.

Der aktive Carbonylkomplex wird dabei in der Regel in situ, d.h. im Hydroformylierungsreaktor, aus einem Salz oder einer Verbindung des Metalls M, dem Liganden und Kohlenmonoxid gebildet; er kann aber auch getrennt hergestellt und als solcher eingesetzt werden.

Die Komplexkatalysatoren bestehen bevorzugt aus einem Zentralatom M, ausgewählt aus den Übergangsmetallen Cobalt, Ruthenium, Rhodium, Palladium oder Platin, insbesondere aber Cobalt und Rhodium, komplexiert mit Carbonyl- sowie Hydrido-, Alkyl- oder Acylresten sowie als Liganden die erfindungsgemäß zu verwendenden ein- oder mehrzähnigen bevorzugten Phosphabenzole. Werden die Komplexkatalysatoren in situ erzeugt, setzt man einfache Precursorkomplexe wie Rhodiumbiscarbonylacetylacetonat oder Rhodiumacetat in Gegenwart der entsprechenden Liganden den Reaktionsbedingungen aus oder man versetzt Precursorkomplexe mit aktivierenden Zusätzen wie z.B. Brönsted- oder Lewissäuren sowie Lewisbasen.

Zur in situ Bildung des Katalysators im Reaktionsgemisch setzt man den Ligand im molaren Verhältnis (gerechnet als Äquivalent Phosphor) zu Rhodium von 1:1 bis 1000:1 ein und verwendet zusätzlich ein inertes Lösungsmittel. Besonders bevorzugte Lösungsmittel sind die Aldehyde, die durch Umsetzung des jeweiligen Olefins entstehen, sowie die syntheseeigenen Hochsieder, die durch Folgereaktionen des jeweiligen Aldehyds im Hydroformylierungsverfahren entstehen. Bei durch geeignete Substituenten hydrophylisierten Liganden werden bevorzugt Wasser, Alkohole oder andere polare Lösungsmittel eingesetzt.

Die Zusammensetzung des im erfindungsgemäßen Hydroformylierungsverfahren eingesetzten Synthesegases $CO/H_2$ kann in weiten Bereichen variiert werden. Beispielsweise kann Synthesegas mit $CO/H_2$-Molverhältnissen von 5:95 bis 70:30 erfolgreich eingesetzt werden, bevorzugt wird Synthesegas mit $CO/H_2$-Verhältnissen von 40:60 bis

60:40, besonders bevorzugt wird ein $CO/H_2$ Verhältnis von etwa 1:1 angewandt.

Die Hydroformylierungsreaktion mit dem neuen Katalysator wird vorzugsweise bei einer Temperatur zwischen 20 und 180°C, insbesondere 50 bis 150°C, durchgeführt. Für jedes Katalysatorsystem wird jedoch zweckmäßig eine optimale Temperatur experimentell ermittelt. Der Reaktionsdruck kann je nach Cokatalysator, d.h. Ligand, und Substrat in einem Bereich von Normaldruck, d.h. Atmosphärendruck, bis 700 bar, vorzugsweise bis 300 bar, schwanken, wobei man normalerweise Reaktionen in einem Bereich bis zu etwa 30 bar als Niederdruck-, in einem Bereich bis zu etwa 100 bar als Mitteldruck- und über 100 bar als Hochdruckreaktion bezeichnet.

Dabei arbeitet man in der Regel mit dem homogen im Reaktionsmedium gelösten Katalysator, der vom Austrag der Hydroformylierungsreakton abgetrennt und in die Hydroformylierungsstufe zurückgeführt wird.

Man erhält in der Regel nahezu ausschließlich die entsprechenden Aldehyde in ausgezeichneten Ausbeuten.

Als erfindungsgemäß zu hydroformylierende Olefine kommen α-Olefine oder interne Olefine oder interne, verzweigte Olefine in Betracht. Beispielsweise seien folgende Olefine genannt:

Ethylen, Propen, 1-Buten, 1-Octen, $C_{5-20}$ α-Olefine, lineare $C_{5-20}$ interne-Olefine, Buten-2; verzweigte, interne Octen-Gemische; verzweigte, interne Nonen-Gemische; verzweigte, interne Dodecen-Gemische, Cyclohexen, α-Pinen, Styrol, 4-Isobutylstyrol, 3-Pentensäuremethylester, 4-Pentensäuremethylester, Ölsäuremethylester, 3-Pentennitril, 4-Pentennitril, 2,7-Octadienol-1, 7-Octenal, Acrylsäuremethylester, Methacrylsäuremethylester, Acrylnitril, Vinylacetat, Vinylglycoldiacetat, Vinylmethylether, Polypropen, Polyisobutylen. Ebenfalls geeignete Substrate sind Di- oder Polyene mit isolierten oder konjugierten Doppelbindungen. Beispiele sind 1,3-Butadien, 1,5-Hexadien, Vinylcyclohexen, Dicylopentadien, 1,5,9-Cyclooctatrien, Butadienhomo- und Copolymere, Polyisobuten.

Darüber hinaus wird die Hydroformylierungsreaktion in an sich bekannter Weise durchgeführt. Einzelheiten der Verfahrensführung sind Beller und Falbe a.a.O. zu entnehmen, deren Angaben als hier inkorporiert gelten sollen.

Beispiele

Ligand 1:

Katalysatorprecursorsynthese: 2,6-Dimethyl-4-Phenylphosphabenzol wurde nach Literaturangaben in zwei Stufen hergestellt:

1. Stufe: Die Synthese des 2,6-Dimethyl-4-Phenylpyryliumperchloratsalzes auf der Basis von Methylstyrol und Essigsäureanhydrid wurde wie in Houben-Weyl, Band 7b, S. 807 ff. beschrieben durchgeführt.

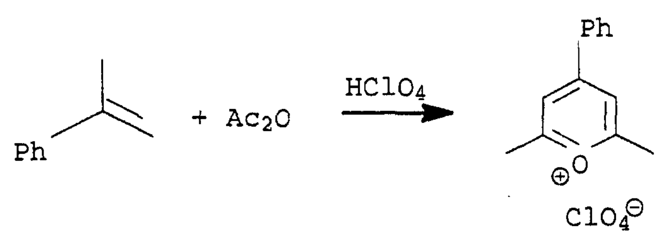


2. Stufe: Anschließend wurde das 2,6-Dimethyl-4-Phenylpyryliumsalz mit Hilfe von $PH_4I$ und Base in 2,6-Dimethyl-4-Phenylphosphabenzol wie in der Dissertation, F. Lieb, Universität Würzburg, 1969, S. 102 beschrieben umgewandelt ($PH_4I$ wurde nach der Vorschrift von Brauer, "Handbuch der präparativen anorganischen Chemie", S. 475 ff, bereitgestellt).

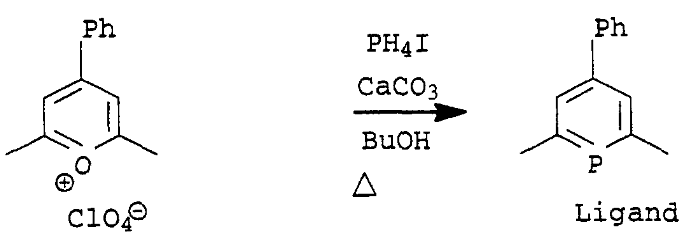


Ligand 1

Ligand 2:

2,3,4,6-Tetraphenylphosphabenzol wurde nach Literaturangaben in zwei Stufen hergestellt:

1. Stufe: Die Synthese des 2,3,4,6-Tetraphenylpyryliumtetrafluoroboratsalzes auf der Basis von 1,3-Diphenyl-3-oxo-propen (Chalkon) und Desoxybenzoin wurde wie in Houben-Weyl, Band E7b, S. 856-857 beschrieben durchgeführt.

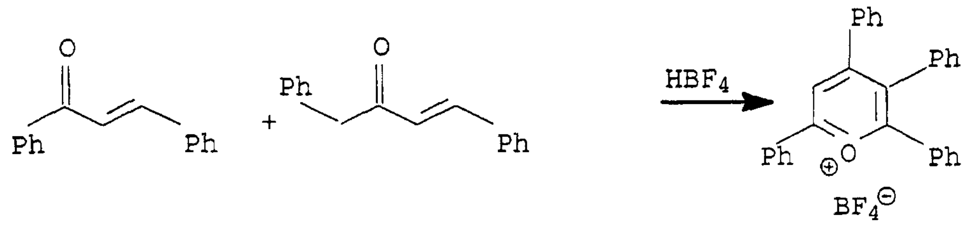


2. Stufe: Anschließend wurde das 2,3,4,6-Tetraphenylpyryliumsalz mit Hilfe von $P(SiMe_3)_3$ in 2,3,4,6-Tetraphenylphosphabenzol (Angew. Chem., 1967, 79, S. 475 ff) umgewandelt.

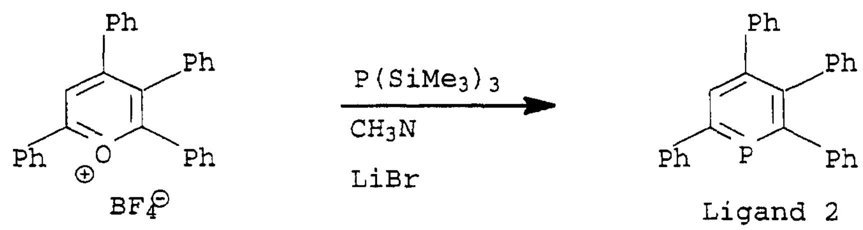


Ligand 2

Ligand 3:

Katalysatorprecursorsynthese: 2,4,6-Triphenylphosphabenzol wurde nach DE-A 1618668 aus dem kommerziell verfügbaren 2,4,6-Triphenylpyryliumtetrafluoroboratsalz (Fa. Aldrich) mit Hilfe von $P(CH_2OH)_3$ hergestellt. $P(CH_2OH)_3$ wurde aus kommerziell verfügbarem $[P(CH_2OH)_4]Cl$ (Aldrich) nach Ellis et al. Inorg. Chem. 1992, 31, 3026-33 hergestellt:

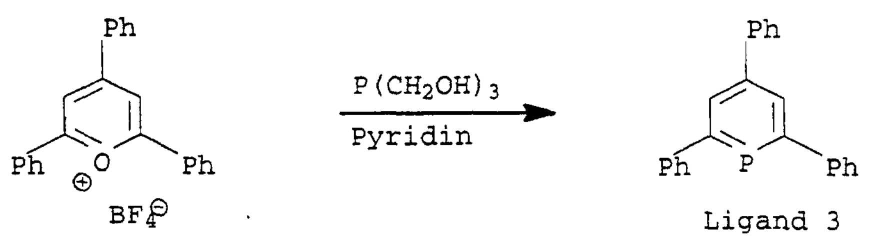


Ligand 3

Ligand 4:

Katalysatorprecursorsynthese: 2,4 -Diphenyl-6(2-naphthyl)phosphabenzol wurde analog zu Ligand 2 in zwei Stufen aus 1,3-Diphenyl-3-oxo-propen (Chalkon) und 2'-Acetonaphthon hergestellt:

1. Stufe:

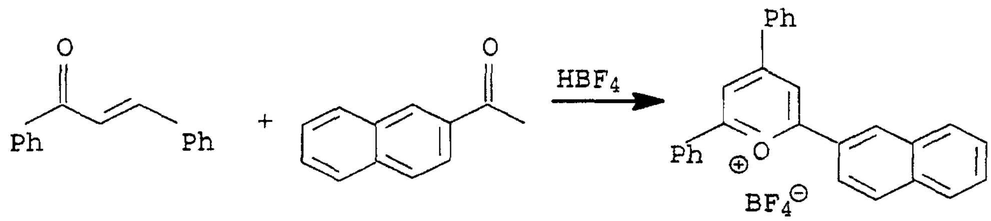


2. Stufe:

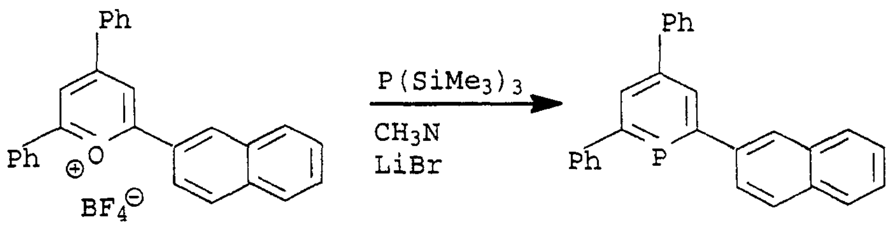


Ligand 4

Ligand 5:

Katalysatorprecursorsynthese: Ligand 5 wurde in sechs Stufen hergestellt:

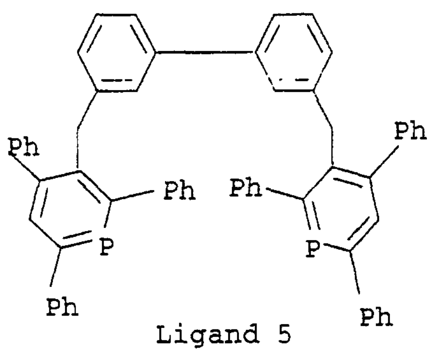

Ligand 5

1. Stufe: Darstellung von 3,3' -Bis-bromomethyl-diphenyl

| | |
|---|---|
| Ansatz: | 25 g = 0,137 mol 3,3'-ditolyl<br>51 g = 0,287 mol N-Bromsuccinimid<br>1 g = 4,13 mmol Di-Benzoylperoxid |
| Lösungsmittel: | Tetrachlorkohlenstoff |

Versuchsdurchführung:

Der Ansatz wurde 5 Stunden unter Rückfluß am Sieden gehalten und anschließend heiß filtriert, um das Succinimid abzutrennen. Aus dem Filtrat wurde das Produkt kristallisiert.

2. Stufe: Darstellung von 3,3'-Bis-jodomethyl-diphenyl

| | |
|---|---|
| Ansatz: | 9 g = 26,5 mmol 3,3'-Bis-bromomethyl-diphenyl 7,94 g = 53 mmol Natriumjodid als 1 molare Lösung in Aceton |
| Lösungsmittel: | Aceton |

Versuchsdurchführung: Das 3,3'-Bis-bromomethyl-diphenyl wurde in Aceton gelöst und zu dieser Lösung langsam die Natriumjodidlösung dosiert. Der Reaktionsansatz wurde bis zur vollständigen Umsetzung gerührt und anschließend das ausgefallene Natriumbromid unter Anwendung verminderten Drucks abfiltriert. Aus dem Filtrat wurde das 3,3'-Bis-jodmethyl-diphenyl kristallisiert. Zur Kristallisation von weiterem Produkt wurde die Mutterlauge mit geringen Mengen Wasser versetzt.

3. Stufe: Darstellung von 1,3,5-Triphenyl-pent-2-en-1,5-dion

| | |
|---|---|
| Ansatz: | 10 g = 25,3 mmol 2,4,6-Triphenylpyryliumtetrafluoroborat 13,7 g = 101,2 mmol Natriumacetattrihydrat |
| Lösungsmittel: | 10 ml Wasser/50 ml Methanol/100 ml Aceton |

Versuchsdurchführung: Das Natriumacetat wurde zusammen mit dem Lösungsmittelgemisch auf 60°C erwärmt, die Heizquelle entfernt und das feste Pyryliumsalz portionsweise in diese Mischung eingetragen. Dabei verfärbte sich das Reaktionsgemisch sofort orange. Die Reaktionsmischung wurde 4 Stunden ohne weitere Wärmezufuhr gerührt. Anschließend wurde dem Reaktionsgemisch Diethylether zugesetzt und die wäßrige Phase mehrmals mit Diethylether gewaschen. Die vereinigten Etherextrakte wurden über Magnesiumsulfat getrocknet, anschließend wurde ein Teil des Lösungsmittels abdestilliert. Aus der so eingeengten Lösung kristallisierte das Produkt 1,3,5-Triphenyl-pent-2-en-1,5-dion (G.W. Fischer et al: J. Prakt. Chem. 326, 287 (1984), insbesondere S. 298).

| | |
|---|---|
| 4. Stufe: | Darstellung von 1,3,5-Triphenyl-pent-2-en-1,5-dion-Natriumenolat |
| Ansatz: | 5 g = 15,38 mmol l,3,5-Triphenyl-pent-2-en-1,5-dion<br>0,89 g = 15,38 mmol Natriummethylat als 30%ige Lösung in Methanol |
| Lösungsmittel: | Benzol, Diethylether |

Versuchsdurchführung: Zu einer Lösung des Natriumenolats des 1,3,5-Triphenyl-pent-2-en-1,5-dions in Benzol wurde langsam die Natriummethylatlösung dosiert. Dann wurden zur Reaktionsmischung 200 ml Diethylether gegeben und die Mischung 1 Stunde unter Ausschluß von Luft gerührt. Das ausgefallene feste Produkt wurde abfiltriert und bei vermindertem Druck getrocknet (G.W. Fischer: J. Prakt. Chem. 327, 983 (1985), insbesondere S. 987).

| | |
|---|---|
| 5. Stufe: | Darstellung von Bis-3,3'(3(2,4,6-triphenylpyryliummethyl)-1,1'-diphenyldiperchlorat |
| Ansatz: | 3,9 g = 11,21 mmol 1,3,5-Triphenyl-pent-2-en-1,5-dion-natriumenolat<br>2,43 g = 5,6 mmol 3,3'-Bis-jodomethyl-diphenyl |

Versuchsdurchführung: Zu einer Lösung des Enolats aus der 4. Stufe wurde bei Raumtemperatur 3,3'-Bis-jodomethyl-diphenyl gegeben und eine Stunde gerührt. Die Reaktionslösung wurde in einem Diethylether/Wasser-Gemisch aufgenommen, die organische Phase mehrmals mit Wasser gewaschen, über Natriumsulfat getrocknet und bei vermindertem Druck eingeengt. Anschließend wurde der Reaktionsaustrag mit Ethanol vermischt, Perchlorsäure hinzugefügt und 6 h unter Rückfluß am Sieden gehalten. Nach dem Erkalten dieser Mischung wurde die Abscheidung des Pyryliumsalzes durch Zugabe von Diethylether vervollständigt, dieses abfiltriert und bei vermindertem Druck getrocknet (G.W. Fischer: J. Prakt. Chem. 327, 983 (1985), insbesondere S. 988).

6. Stufe: Darstellung von Ligand 5

Eine Lösung von 2,5 g des in Stufe 5 erhaltenen Pyryliumsalzes in Ethanol wurde mit 0,5 g einer 30gew. %igen Lösung von Bromwasserstoff in Eisessig (Aldrich) vermischt und in einem Druckbehälter mit Phosphorwasserstoff ($PH_3$) umgesetzt. Hierzu wurden bei Raumtemperatur Phosphorwasserstoffgas bis zum Erreichen eines Drucks von 5 bar in den Druckbehälter eingepreßt, anschließend der befüllte Druckbehälter 4 Stunden auf 110°C erwärmt und dabei ein Druck von 12,5 bar eingestellt. Nach Abkühlung und Entspannung wurde das ausgefallene Produkt abfiltriert und umkristallisiert. Aus der Lösung konnte weiteres Produkt isoliert werden.

Allgemeine Versuchsbeschreibung:

Alle Versuche (diskontinuierlich) wurden in einem 100 ml Autoklav (Material HC) durchgeführt. Das Reaktionsgemisch wurde 10 min auf Reaktionstemperatur erhitzt und die Lösung wurde mit einem Begasungsrührer kräftig gerührt. Mittels $CO/H_2$ (1:1) wurde der gewünschte Druck eingestellt. Während der Reaktion wurde der Druck im Reaktor durch Nachpressen über einen Druckregler auf dem gewünschten Druckniveau gehalten. Nach der Reaktionszeit wurde der Autoklav abgekühlt, entspannt und entleert. Eine Analyse des Reaktionsgemisches wurde mittels GC - mit internem Standard und Korrekturfaktoren - durchgeführt.

Beispiel 1: Niederdruck-Hydroformylierung von Octen-1

3,1 mg Rhodiumbiscarbonylacetylacetonat (0,012 mmol), 23 mg Ligand 1 (0,12 mmol), 5,8 g Octen-1 (52 mmol) und 6,0 ml Texanol® wurden auf 100°C erhitzt. Mittels $CO/H_2$ (1:1) wurde der gewünschte Druck von 10 bar eingestellt. Nach 4 h wurde der Autoklav abgekühlt, entspannt und entleert. Eine Analyse des Reaktionsgemisches wurde mittels GC - mit internem Standard und Korrekturfaktoren - durchgeführt. Der Umsatz an Octen-1 betrug 100 %, die Ausbeute an Nonanalen 92 %, die Selektivität zu Nonanalen 92,5 % (n-Anteil von 59,2 %), die Selektivität zu internen Olefinen 2,7 %.

Beispiel 2: Mitteldruck-Hydroformylierung von Octen-N

Die Aldehyde im Reaktionsgemisch von Beispiel 1 wurden bei 100°C unter Vakuum abdestilliert. Zurück blieb eine homogene Lösung, bestehend aus Aktivkatalysator, Texanol® und reaktionseigenen Hochsiedern. Dazu wurden 18 g Octen-N (Isooctengemisch, Verzweigungsgrad 1,3) unter Luftausschluß zugegeben. Das Gemisch wurde auf 130°C erhitzt und mittels $CO/H_2$ (1:1) der gewünschte Druck von 60 bar eingestellt. Nach 4 h wurde der Autoklav abgekühlt, entspannt und entleert. Eine Analyse des Reaktionsgemisches wurde mittels GC - mit internem Standard und Korrekturfaktoren - durchgeführt. Der Octenumsatz betrug 63 %, die Selektivität zu Nonanalisomeren 100 %.

Beispiel 3: Mitteldruck-Hydroformylierung von Styrol

0,375 mol-% Rhodiumbiscarbonylacetylacetonat, Ligand 1 in einem Rhodium-zu-Ligand-Molverhältnis von 1:5, Styrol und Toluol als Lösungsmittel wurden bei 20°C in einem Autoklaven mit $CO/H_2$ (1:1) bei einem Druck von 50 bar umgesetzt. Nach 22 h wurde der Autoklav entspannt und entleert. Eine Analyse des Reaktionsgemisches wurde mittels GC - mit internem Standard und Korrekturfaktoren - durchgeführt. Der Styrolumsatz betrug 51 %, das Verhältnis von verzweigtem (2-Phenylpropanal) zu linearem (3-Phenylpropanal) Produkt 22,3:1.

Beispiel 4: Mitteldruck-Hydroformylierung von Styrol

0,375 mol-% Rhodiumbiscarbonylacetylacetonat, Ligand 1 in einem Rhodium-zu-Ligand-Molverhältnis von 1:2, Styrol und Toluol als Lösungsmittel wurden bei 20°C in einem Autoklaven mit $CO/H_2$ (1:1) bei einem Druck von 50 bar umgesetzt. Nach 22 h wurde der Autoklav entspannt und entleert. Eine Analyse des Reaktionsgemisches wurde mittels GC - mit internem Standard und Korrekturfaktoren - durchgeführt. Der Styrolumsatz betrug 80 %, das Verhältnis von verzweigtem (2-Phenylpropanal) zu linearem (3-Phenylpropanal) Produkt 26,6:1.

Beispiel 5: Niederdruck-Hydroformylierung von Octen-1

3,1 mg Rhodiumbiscarbonylacetylacetonat (0,012 mmol), 26 mg Ligand 2 (0,065 mmol), 5,6 g Octen-1 (50 mmol) und 6,0 ml Texanol® wurden auf 100°C erhitzt. Mittels $CO/H_2$ (1:1) wurde der gewünschte Druck von 10 bar eingestellt. Nach 4 h wurde der Autoklav abgekühlt, entspannt und entleert. Eine Analyse des Reaktionsgemisches wurde mittels GC - mit internem Standard und Korrekturfaktoren - durchgeführt. Der Umsatz an Octen-1 betrug 100

%, die Ausbeute an Nonanalen 88 %, die Selektivität zu Nonanalen 88 % (Aldehydzusammensetzung: 15 % 2-Propylhexanal, 19 % 2-Ethylheptanal, 38 % Methyloctanal, 28 % n-Nonanal; n-Anteil von 28 %), die Selektivität zu internen Olefinen 12 %.

Beispiel 6: Mitteldruck-Hydroformylierung von Octen-N

Die Aldehyde im Reaktionsgemisch von Beispiel 5 wurden bei 100°C unter Vakuum abdestilliert. Zurück blieb eine homogene Lösung bestehend aus Aktivkatalysator, Texanol® und reaktionseigenen Hochsiedern. Dazu wurden 5,3 g Octen-N (Isooctengemisch, Verzweigungsgrad 1,3) unter Luftausschluß zugegeben. Das Gemisch wurde auf 130°C erhitzt und mittels $CO/H_2$ (1:1) der gewünschte Druck von 60 bar eingestellt. Nach 4 h wurde der Autoklav abgekühlt, entspannt und entleert. Eine Analyse des Reaktionsgemisches wurde mittels GC - mit internem Standard und Korrekturfaktoren - durchgeführt. Der Octenumsatz betrug 56 %, die Selektivität zu Nonanalisomeren 100 %.

Beispiel 7: Mitteldruck-Hydroformylierung von Octen-N

Die Aldehyde im Reaktionsgemisch von Beispiel 6 wurden bei 100°C unter Vakuum abdestilliert. Zurück blieb eine homogene Lösung bestehend aus Aktivkatalysator, Texanol® und reaktionseigenen Hochsiedern. Dazu wurden 5,3 g Octen-N (Isooctengemisch, Verzweigungsgrad 1,3) unter Luftausschluß zugegeben. Das Gemisch wurde auf 130°C erhitzt und mittels $CO/H_2$ (1:1) der gewünschte Druck von 60 bar eingestellt. Nach 4 h wurde der Autoklav abgekühlt, entspannt und entleert. Eine Analyse des Reaktionsgemisches wurde mittels GC - mit internem Standard und Korrekturfaktoren - durchgeführt. Der Octenumsatz betrug 55 %, die Selektivität zu Nonanalisomeren 100 %.

Beispiel 8: Mitteldruck-Hydroformylierung von Styrol

0,375 mol-% Rhodiumbiscarbonylacetylacetonat, Ligand 2 in einem Rhodium-zu-Ligand-Molverhältnis von 1:5, Styrol (0,65 mol/1) und Toluol als Lösungsmittel wurden bei 20°C in einem Autoklaven mit $CO/H_2$ (1:1) bei einem Druck von 50 bar umgesetzt. Nach 22 h wurde der Autoklav entspannt und entleert. Eine Analyse des Reaktionsgemisches wurde mittels GC- mit internem Standard und Korrekturfaktoren - durchgeführt. Der Styrolumsatz betrug 98 %, das Verhältnis von verzweigtem (2-Phenylpropanal) zu linearem (3-Phenylpropanal) Produkt 18:1.

Beispiel 9: Mitteldruck-Hydroformylierung von Octen-N

Es wurde, wie in Beispiel 1 beschrieben, ein Versuch zur Hydroformylierung von Octen-N mit Ligand 3 (51 ppm Rh, L/Rh-Molverhältnis = 10,8) durchgeführt. Der Druck (60 bar), die Temperatur (130°C) sowie die Reaktionszeit (4 h) wurden wie in Beispiel 2 eingestellt. Der Katalysator wurde, analog zu Beispiel 2, abgetrennt und vier Mal wiedereingesetzt. Folgende Ausbeuten wurden bei annähernd 100%iger Selektivität zu Nonanalen erzielt:

| Versuch | Ausbeute Nonanale % |
|---|---|
| Ersteinsatz | 63 |
| Recycle 1 | 56 |
| Recycle 2 | 53 |
| Recycle 3 | 55 |
| Recycle 4 | 52 |

Beispiel 10: Niederdruck-Hydroformylierung von Octen-1

Es wurde, wie in Beispiel 1 beschrieben, Versuche zur Hydroformylierung von Octen-1 mit Ligand 3 bei 90°C durchgeführt. Abweichend wurde das Ligand-zu-Rhodium-Verhältnis variiert.

| Rh-Gehalt | L/Rh | Octen-1 Umsatz | Nonanal-ausbeute | Bildung interner Olefine: Selektivität | Nonanal-Selektivität | n-Anteil | α-Anteil |
|---|---|---|---|---|---|---|---|
| ppm | mol/mol | % | % | % | % | % | % |
| 51 | 21 | 99 | 64 | 35,6 | 64,4 | 40,8 | 79,6 |
| 60 | 35 | 99 | 84 | 16,9 | 84,9 | 49,2 | 86,7 |
| 60 | 89 | 98 | 90 | 8,5 | 92,1 | 65,2 | 99,6 |

n-Anteil = (1-Nonanal/Gesamtaldehyde) x 100
α-Anteil = [(1-Nonanal + 2-Methyloctanal)/Gesamtaldehyd] x 100

Beispiel 11: Niederdruckhydroformylierung von Octen-1

Es wurden, wie in Beispiel 1 beschrieben, Versuche zur Hydroformylierung von Octen-1 mit Ligand 3 bei einem Ligand-zu-Rhodium-Verhältnis von 11 durchgeführt. Zusätzlich wurde die Temperatur variiert.

| Rh-Gehalt | Temperatur | Octen-1 Umsatz | Nonanalausbeute | n-Anteil | interne Olefine Selektivität | Nonanal-Selektivität |
|---|---|---|---|---|---|---|
| ppm | °C | % | % | % | % | % |
| 52 | 100 | 99 | 69 | 31,4 | 29,3 | 69,1 |
| 58 | 90 | 98 | 12 | 50,7 | 81,7 | 11,9 |
| 51 | 80 | 93 | 8 | 67,7 | 82,3 | 8,4 |

Beispiel 12: Niederdruck-Hydroformylierung von Cyclohexen

0,375 mol-% Rhodiumbiscarbonylacetylacetonat und Ligand 3 in einem Rhodium-zu-Ligand-Molverhältnis von 1:10, Cyclohexen (0,68 mol/l; Cyclohexen/Rh = 730 mol/mol) und Toluol als Lösungsmittel wurden bei 90°C in einem Autoklaven mit $CO/H_2$ (1:1) bei einem Druck von 20 bar umgesetzt. Nach 1 h wurde der Autoklav entspannt unt entleert. Eine Analyse des Reaktionsgemisches wurde mittels GC - mit internem Standard und Korrekturfaktoren - durchgeführt.

Die Ausbeute an Cyclohexancarbaldehyd betrug 29 %, d.h. die Umsatzzahl (TOF: Turnover Frequence) betrug 214 $h^{-1}$. Die Umsatzzahl TOF errechnet sich nach der Formel:

$$TOF = \frac{\text{mol Produkt}}{\text{mol Katalysator} \cdot h}$$

Beispiel 13: Hydroformylierung von Octen-N

261 mg Dicobaltoctacarbonyl (0,763 mmol), 2,16 g Ligand 3 (6,9 mmol), 30 g Octen-N (267,4 mmol) und 10 g Toluol wurden bei 30 bar $CO/H_2$(1:1)-Druck auf 150°C erhitzt. Durch Aufpressen von weiterem $CO/H_2$(1:1) wurde der Druck auf 50 bar eingestellt. Nach 5 Stunden wurde der Autoklav abgekühlt, entspannt und entleert. Eine Analyse des Reaktionsgemisches wurde mittels GC (mit internem Standard und Korrekturfaktoren) durchgeführt.

Der Umsatz an Octen-N betrug 9,8 %, die Ausbeute an $C_9$-Aldehyden 8,3 %, die Selektivität zu Aldehyden 85 % (n-Anteil 5,5, %).

Beispiel 14: Hydroformylierung von Octen-1

90 mg Dicobaltoctacarbonyl (0,263 mmol), 0,41 g Ligand 3 (1,315 mmol), 30 g Octen-1 (267,4 mmol) und 10 g Toluol wurden bei 30 bar $CO/H_2$(1:1) auf 130°C erhitzt. Durch Aufpressen von weiterem $CO/H_2$(1:1) wurde der gewünschte Druck von 75 bar eingestellt. Nach 9 Stunden wurde der Autoklav abgekühlt, entspannt und entleert. Eine Analyse des Reaktionsgemisches wurde mittels GC (mit internem Standard und Korrekturfaktoren) durchgeführt.

Der Umsatz an Octen-1 betrug 98,9 %, die Ausbeute an Nonanalen 70,7 %, die Selektivität zu Nonanalen 71,5 % (n-Anteil 45,3 %), die Selektivität zu internen Olefinen 11,1 %.

Beispiel 15: Hydroformylierung von internen Dodecenen

261 mg Dicobaltoctacarbonyl (0,763 mmol), 2,35 g Ligand 3 (7,5 mmol), 30 g interne Dodecene (178,2 mmol) und 10 g Toluol wurden bei 30 bar $CO/H_2$(1:1) auf 150°C erhitzt. Durch Aufpressen von zusätzlichem $CO/H_2$(1:1) wurde der Druck auf 50 bar eingestellt. Nach 5 Stunden wurde der Autoklav abgekühlt, entspannt und entleert. Eine Analyse des Reaktionsgemisches wurde mittels GC (mit internem Standard und Korrekturfaktoren) durchgeführt.

Der Umsatz an Dodecen betrug 26,2 %, die Ausbeute an Tridecanalen 16,7 %, die Selektivität zu Tridecanalen 53,3 % (n-Anteil 0,27 %).

Beispiel 16: Hydroformylierung von Isobuten

31,2 mg Rhodiumbiscarbonylacetylacetonat (0,12 mmol) und Ligand 3 in einem Rhodium-zu-Ligand-Molverhältnis von 1:15, 17,2 g Isobuten (0,31 mol) und 50 ml Texanol® als Lösungsmittel wurden bei 110°C in einem Autoklaven von $CO/H_2$(1:1) bei einem Druck von 50 bar umgesetzt. Nach 5 Stunden wurde der Autoklav entspannt und entleert. Eine Analyse des Reaktionsgemisches wurde mittels GC - mit internem Standard und Korrekturfaktoren - durchgeführt.

Der Isobutenumsatz betrug 100 %, die Pentanalausbeute betrug 93,5 %, das Verhältnis von verzweigtem (Pivalaldehyd) zu linearem (Isovaleraldehyd) Produkt betrug 1:600.

Vergleichsbeispiel 1: Hydroformylierung von Isobuten und Rhodium/ Triphenylphosphan

31,2 mg Rhodiumbiscarbonylacetylacetonat (0,12 mmol) und Triphenylphosphan in einem Rhodium-zu-Triphenylphosphan-Molverhältnis von 1:10, 14,5 g Isobuten (0,26 mol) und 50 ml Texanol® als Lösungsmittel wurden bei 110°C in einem Autoklaven mit $CO/H_2$(1:1) bei einem Druck von 50 bar umgesetzt. Nach 5 Stunden wurde der Autoklav entspannt und entleert. Eine Analyse des Reaktionsgemisches wurde mittels GC - mit internem Standard und Korrekturfaktoren - durchgeführt.

Der Isobutenumsatz betrug 82 %, die Pentanalausbeute betrug 69 %, das Verhältnis von verzweigtem (Pivalaldehyd) zu linearem (Isovaleraldehyd) Produkt betrug 1:300.

Beispiel 17: Hydroformylierung von Isoprenol

37 mg Rhodiumbiscarbonylacetylacetonat (0,14 mmol) und Ligand 3 in einem Rhodium-zu-Ligand-Molverhältnis von 1:15 und 75 g Isoprenol (0,87 mol) wurden bei 90°C in einem Autoklaven mit $CO/H_2$(1:1) bei einem Druck von 50 bar umgesetzt. Nach 5 Stunden wurde der Autoklav entspannt und entleert. Eine Analyse des Reaktionsgemisches wurde mittels GC-Analyse durchgeführt.

Der Isoprenol-Umsatz betrug 100 %, das Produkt bestand aus 79,2 % 2-Hydroxy-4-methyltetrahydropyran, 6,5 % 3-Methylbutanol, 1,2 % 3-Methylbutanal, 3,2 % 4-Methyl-3,6-dihydro-2H-pyran (Hauptkomponenten).

Beispiel 18: Hydroformylierung von Polyisobuten

20 mg Rhodiumbiscarbonylacetylacetonat (0,077 mmol) und Ligand 3 in einem Rhodium-zu-Ligand-Molverhältnis von 1:15, 40 g Polyisobuten der mittleren Molmasse ($M_n$) 1000, 40 g Mihagol als Lösungsmittel wurden bei 150°C in einem Autoklaven mit $CO/H_2$(1:1) bei einem Druck von 50 bar umgesetzt. Nach 5 Stunden wurde der Autoklav entspannt und entleert. Eine Analyse des Reaktionsproduktes mittels $^{13}C$-NMR-Spektroskopie ergab eine Polyisobutenaldehyd-Ausbeute von 64 %.

Beispiel 19: Niederdruck-Hydroformylierung von Styrol

0,357 mol-% Rhodiumbiscarbonylacetylacetonat, Ligand 4 in einem Rhodium-zu-Ligand-Molverhältnis von 1:2, Styrol (0,65 mol/l) und Toluol als Lösungsmittel wurden bei 20°C in einem Autoklaven $CO/H_2$(1:1) bei einem Druck von 20 bar umgesetzt. Nach 22 h wurde der Autoklav entspannt und entleert. Eine Analyse des Reaktionsgemisches wurde mittels GC - mit internem Standard und Korrekturfaktoren - durchgeführt.

Die Aldehydausbeute betrug 99 %, das Verhältnis von verzweigtem (2-Phenylpropanal) zu linearem (3-Phenylpropanal) Produkt 26:1.

Beispiel 20: Niederdruck-Hydroformylierung von Octen-1

1,6 mg Rhodiumbiscarbonylacetylacetonat (0,006 mmol), 76 mg Ligand 5 (0,09 mmol; L/Rh-Molverhältnis = 15), 6,1 g Octen-1 (54 mmol) und 6,0 ml Texanol® wurden auf 90°C aufgeheizt. Durch Aufpressen von zusätzlichem CO/H2(1:1) wurde der Druck auf 10 bar eingestellt. Nach 4 h wurde der Autoklav abgekühlt, entspannt und entleert. Eine Analyse des Reaktionsgemisches wurde mittels GC - mit internem Standard und Korrekturfaktoren - durchgeführt.
Der Umsatz an Octen-1 betrug 99 %, die Selektivität zu Nonanalen 64 %, der n-Anteil 71 %, der α-Anteil 98,2 % und die Selektivität zu internen Olefinen 32 %; n-Anteil = (1-Nonanal/Gesamtaldehyde) x 100; α-Anteil = [(1-Nonanal + 2-Methyloctanal)/Gesamtaldehyd] x 100.

**Patentansprüche**

1. Verfahren zur Herstellung von Aldehyden durch Hydroformylierung von Olefinen mit $CO/H_2$ in Gegenwart von Phosphorverbindungen als Liganden enthaltenden Komplexen von Übergangsmetallen der Nebengruppe VIII des Periodischen Systems der Elemente bei Temperaturen von 20 bis 200°C und Drücken von Atmosphärendruck bis 700 bar, dadurch gekennzeichnet, daß man als Liganden Phosphaaromaten mit 5 bis 100 C-Atomen verwendet.

2. Verfahren gemäß Anspruch 1, dadurch gekennzeichnet, daß man als Liganden gegebenenfalls substituiertes Phosphabenzol der Formel I verwendet,

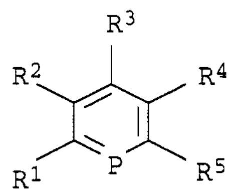

I,

in der $R^1$ - $R^5$ unabhängig voneinander für Wasserstoff, Alkyl-Aralkyl- oder Arylreste oder heterocyclische Reste mit jeweils 1 - 12 C-Atomen steht, ferner für die Reste $-COO^{\ominus}M^{\oplus}$, $-SO_3^{\ominus}M^{\oplus}$ ,

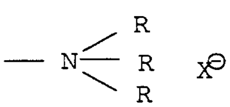

-OR, $-NR_2$, -COOR, -SR, $-(CHRCH_2O)_xR$ und $(CH_2CH_2NR)_x$, wobei R gleiche oder verschiedene Reste ausgewählt aus Wasserstoff oder aliphatischen oder aromatischen Resten mit 1 bis 18 C-Atomen $M^{\oplus}$ ein Kation und $X^{\ominus}$ ein Anion und x die Zahlen 1 bis 120 bedeuten und wobei die Reste $R^1$ - $R^5$ zu annellierten Ringen verbunden sein können.

3. Verfahren gemäß Anspruch 2, dadurch gekennzeichnet, daß man als Liganden Phosphabenzol der Formel I gemäß Anspruch 2 verwendet, das in mindestens einer Seitenkette eine Phosphin- oder Phosphitgruppierung aufweist.

4. Verfahren gemäß Anspruch 1, dadurch gekennzeichnet, daß man als Liganden eine zwei- oder mehrzähnige Verbindung der Formel II verwendet

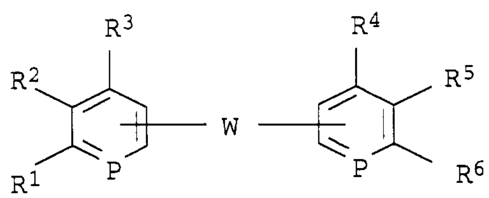

II,

in der $R_1$-$R_6$ die für $R_1$-$R_5$ in Anspruch 2 angegebenen Bedeutungen haben und W eine Brücke in Form einer

kovalenten Bindung oder einer Kette aus 1 bis 10 Atomen bedeutet, die Bestandteil einer cyclischen oder aromatischen Verbindung sein kann und wobei die Phosphaaromaten in ortho- oder meta-Stellung zum Phosphor verknüpft sind, wobei die nicht für die Brücke verwendeten ortho- oder meta-Positionen durch Reste $R^1$ bis $R^5$ gemäß Anspruch 2 substituiert sind, und wobei einer oder mehrere der Substituenten $R^1$ bis $R^6$ ein über eine weitere Brücke W gebundenes Phosphabenzol der Formel I gemäß Anspruch 2 bedeuten können.

**5.** Zweizähnige Phosphabenzole der allgemeinen Formel IIa

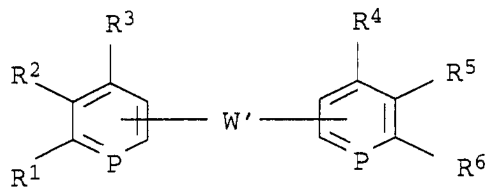

IIa,

in der die Reste $R^1$ bis $R^6$ gleich oder verschieden sind und die in Anspruch 2 für die Reste $R^1$ bis $R^5$ genannte Bedeutung haben und in der W' eine 2,2'-, 4,4'- oder 3,3'-Diphenyldiyl-Gruppe, eine 2,2'- 4,4'- oder 3,3'-Diphenylether-diylgruppe, eine 2,2'-, 4,4'- oder 3,3'-Diphenylmethan-diyl-, eine 2,2'-4,4'- oder 3,3'-Diphenylethan-diyl-gruppe oder eine 2,2'-, 4,4' - oder 3,3' -Diphenylpropan-diyl-Gruppe oder eine Ferrocen-diyl-Gruppe ist, die mit den beiden Phosphabenzolgruppen in ortho- oder meta-Stellung zu deren Phosphoratom jeweils über eine kovalente Bindung, eine Oxa-Gruppe und/oder eine $C_1$- bis $C_4$-Alkylgruppe, insbesondere eine Methylengruppe, verknüpft ist, worin die Diphenyl-, Diphenylether- oder $C_1$- bis $C_3$-Diphenylalkan-Gruppierung unsubstituiert oder mit 1 bis 4 gleichen oder verschiedenen $C_1$- bis $C_4$-Alkylgruppen substituiert ist, und wobei die nicht für die Brücke verwendeten ortho- oder meta-Positionen durch Reste $R^1$ bis $R^5$ gemäß Anspruch 2 substituiert sind, ausgenommen die Verbindungen der Formeln VI

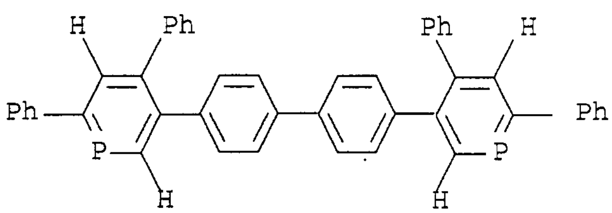

VI

und VII

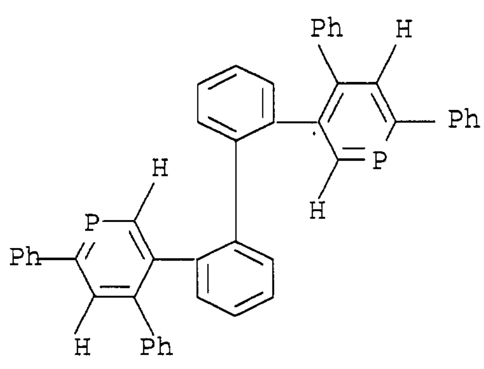

VII

in denen Ph für die Phenylgruppe steht.

**6.** Das zweizähnige Phosphabenzol gemäß Anspruch 5 der Formel IV

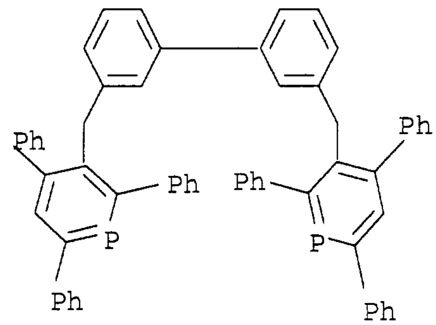

IV,

in der Ph für die Phenylgruppe steht.

**7.** Verfahren gemäß Anspruch 1, dadurch gekennzeichnet, daß man als Liganden ein zweizähniges Phosphabenzol der allgemeinen Formel IIa

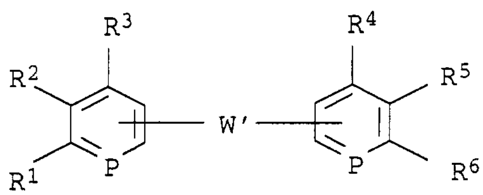

IIa,

verwendet, in der $R^1$ bis $R^6$ und W' die in Anspruch 5 genannte Bedeutung haben.

**8.** Verfahren gemäß Anspruch 1, dadurch gekennzeichnet, daß man als Ligand das zweizähnige Phosphabenzol der Formel IV

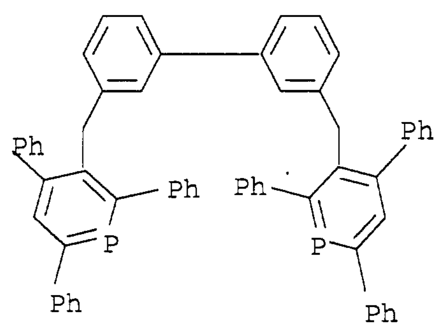

IV,

verwendet.

**9.** Katalysatoren der Formel III

$$M(L)_n(CO)_m \quad III,$$

in der M mindestens ein Zentralatom eines Elements der VIII. Nebengruppe des periodischen Systems der Elemente, L mindestens ein Ligand der Formeln I, II oder IIa oder IV bedeutet

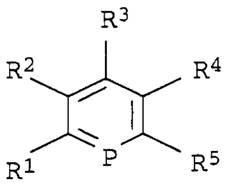 I

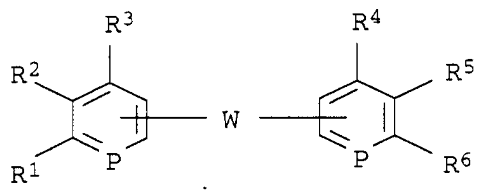 II,

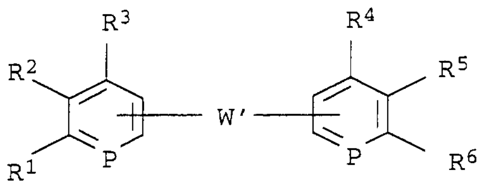 IIa,

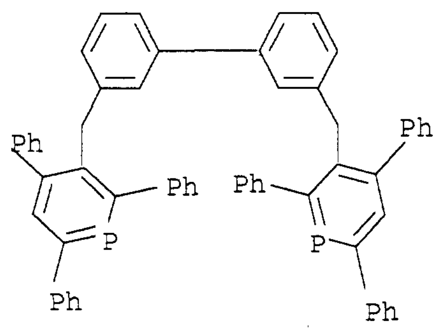 , IV,

in denen $R^1$-$R^6$, W und W' die in Anspruch 2, 4 und 5 angegebenen Bedeutungen haben, n und m jeweils mindestens 1 ist und wobei noch weitere Reste, wie Hydrido, Alkyl- oder Acylreste im Komplex enthalten sein können.

**10.** Katalysatoren gemäß Anspruch 9, dadurch gekennzeichnet, daß sie in Hydroformylierungs- oder Carbonylierungsreaktionen in situ hergestellt sind.

**11.** Verfahren zur Herstellung von Bis-3,3' (3(2,4,6-triphenylphosphabenzol)-1,1' -diphenyl der Formel IV

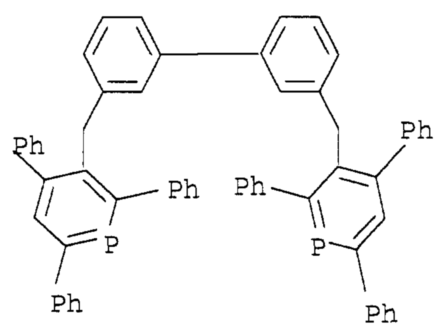 IV,

dadurch gekennzeichnet, daß man das Pyryliumsalz der Formel V

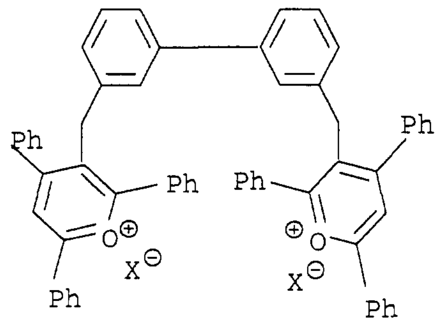

V,

in der $X^{\ominus}$ für das Äquivalent eines Anions steht, mit Phosphorwasserstoff im Temperaturbereich von Raumtemperatur bis 150°C in Gegenwart einer katalytischen Menge Säure bei erhöhtem Druck in An- oder Abwesenheit eines Lösungsmittels in einem Druckbehälter umsetzt.

**12.** Verfahren gemäß Anspruch 11, dadurch gekennzeichnet, daß man während der Umsetzung einen Phosphorwasserstoffpartialdruck im Reaktor von 0,5 bis 50 bar einstellt.

**13.** Verfahren gemäß den Ansprüchen 11 und 12, dadurch gekennzeichnet, daß man den Phosphorwasserstoff bei Raumtemperatur mit dem Pyryliumsalz V in Kontakt bringt, anschließend diese Mischung auf eine Temperatur von über 100°C erhitzt und nach Erreichen dieser Temperatur den Phosphorwasserstoffpartialdruck im Druckbehälter während der Umsetzung durch Einleiten von zusätzlichem Phosphorwasserstoffs im wesentlichen auf einem Druckniveau von 0,5 bis 50 bar hält.

**14.** 2,4-Diphenyl-6 (2-naphthyl)phosphabenzol.

## Claims

**1.** A process for preparing aldehydes by hydroformylation of olefins with $CO/H_2$ in the presence of complexes of transition metals of group VIII of the Periodic Table of the Elements containing phosphorus compounds as ligands at from 20 to 200°C and pressures of from atmospheric pressure to 700 bar, wherein the ligands used are phosphaaromatics having from 5 to 100 carbon atoms.

**2.** A process as claimed in claim 1, wherein the ligands used are unsubstituted or substituted phosphabenzene of the formula I,

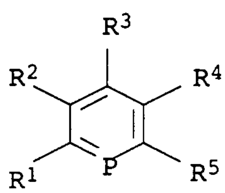

I,

where $R^1$ - $R^5$ are, independently of one another, hydrogen, alkyl, aralkyl or aryl radicals or heterocyclic radicals each having 1-12 carbon atoms, or radicals $-COO^{\ominus}M^{\oplus}$, $-SO_3^{\ominus}M^{\oplus}$ ,

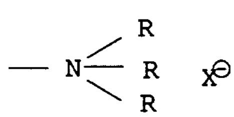

-OR, $-NR_2$, -COOR, -SR, $-(CHRCH_2O)_xR$ and $(CH_2CH_2NR)_x$, where R are identical or different radicals selected from the group consisting of hydrogen or aliphatic or aromatic radicals having from 1 to 18 carbon atoms, $M^{\oplus}$ is a

cation and $X^{\ominus}$ is an anion and x is from 1 to 120, and the radicals $R^1$ - $R^5$ can be joined to form fused rings.

**3.** A process as claimed in claim 2, wherein the ligands used are phosphabenzene of the formula I as set forth in Claim 2 which has a phosphine or phosphite group in at least one side chain.

**4.** A process as claimed in claim 1, wherein the ligands used are a bidentate or multidentate compound of the formula II

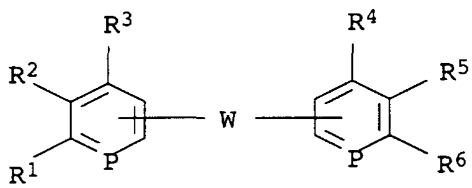
II,

where $R^1$-$R^6$ are as defined for $R^1$-$R^5$ in claim 2 and W is a bridge in the form of a covalent bond or a chain comprising from 1 to 10 atoms which can be part of a cyclic or aromatic compound, and the phosphaaromatics are linked in the ortho or meta position relative to the phosphorus, with the ortho or meta positions not used for the bridge being substituted by radicals $R^1$ to $R^5$ as in claim 2, and one or more of the substituents $R^1$ to $R^6$ can be a phosphabenzene of the formula I as set forth in claim 2 bonded via a further bridge W.

**5.** A bidentate phosphabenzene of the formula IIa

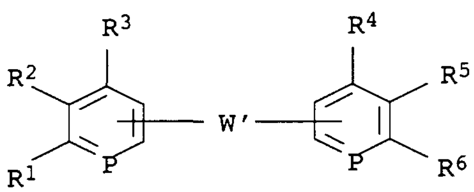
IIa,

where the radicals $R^1$ to $R^6$ are identical or different and are as defined in claim 2 for the radicals $R^1$ to $R^5$ and where W' is a 2,2'-, 4,4'- or 3,3'-biphenyldiyl group, a 2,2'-, 4,4'-or 3,3'-(diphenyl ether)-diyl group, a 2,2'-, 4,4'- or 3,3'-diphenylmethane-diyl group, a 2,2'-, 4,4'- or 3,3'-diphenylethane-diyl group or a 2,2'-, 4,4'- or 3,3'-diphenylpropane-diyl group or a ferrocenediyl group which in each case is linked to the two phosphabenzene groups in the ortho or meta position relative to the phosphorus atom via a covalent bond, an oxa group and/or a $C_1$-$C_4$-alkylene group, in particular a methylene group, where the biphenyl, diphenyl ether or $C_1$-$C_3$-diphenylalkane group is unsubstituted or substituted by from 1 to 4 identical or different $C_1$-$C_4$-alkyl groups, and where the ortho or meta positions not used for the bridge are substituted by radicals $R^1$ to $R^5$ as in claim 2, excluding the compounds of the formula VI

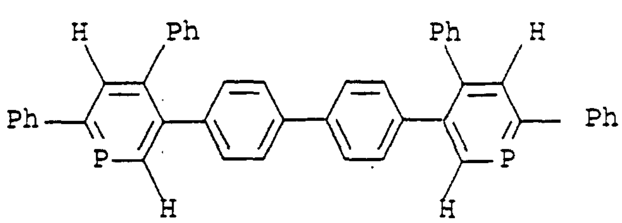
VI

and VII

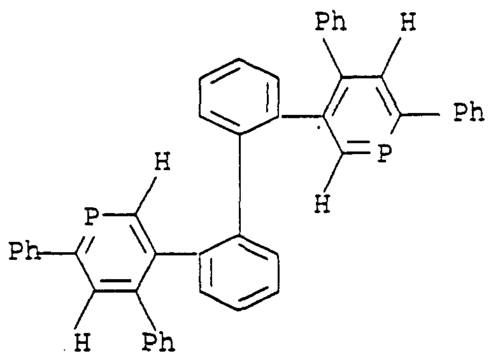

VII

where Ph is the phenyl group.

**6.** A bidentate phosphabenzene as claimed in claim 5 having the formula IV

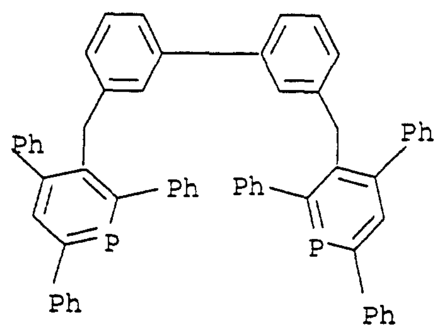

IV,

where pH is the phenyl group.

**7.** A process as claimed in claim 1, wherein the ligands used are a bidentate phosphabenzene of the formula IIa

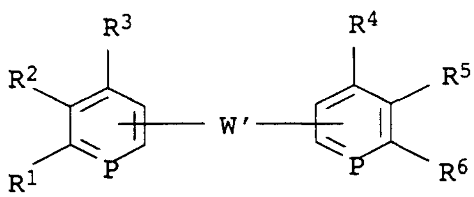

IIa,

where $R^1$ to $R^6$ and W' are as defined in claim 5.

**8.** A process as claimed in claim 1, wherein the ligand used is the bidentate phosphabenzene of the formula IV

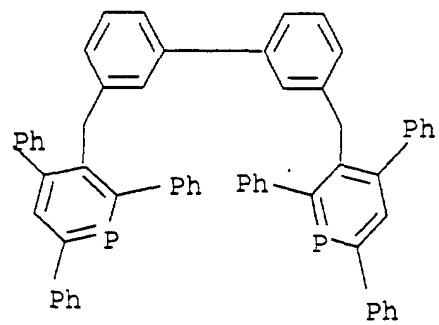

IV.

**9.** A catalyst of the formula III

$$M(L)_n(CO)_m \qquad III,$$

where M is at least one central atom of an element of transition group VIII of the Periodic Table of the Elements, L is at least one ligand of the formula I or II or IIa or IV

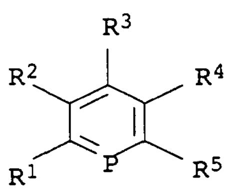

I

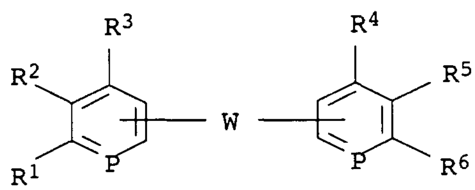

II,

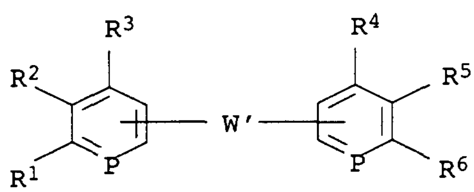

IIa,

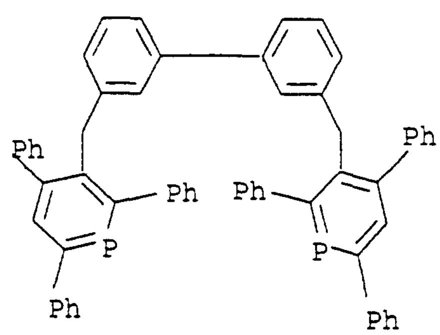

,

IV,

where $R^1$-$R^6$, W and W' are as defined in claims 2, 4 and 5, n and m are each at least 1 and where further radicals such as hydrido, alkyl or acyl radicals may be additionally present in the complex.

**10.** A catalyst as claimed in claim 9 which is prepared in situ in a hydroformylation or carbonylation reaction.

**11.** A process for preparing bis-3,3'-(3-(2,4,6-triphenyl)phosphabenzene)-1,1'-biphenyl of the formula IV

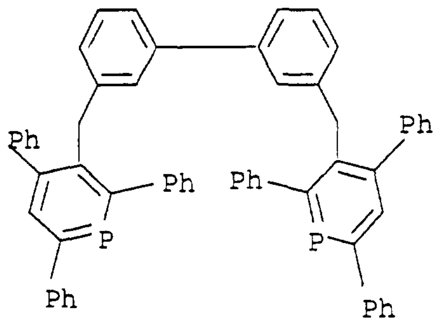

IV,

which comprises reacting the pyrylium salt of the formula V

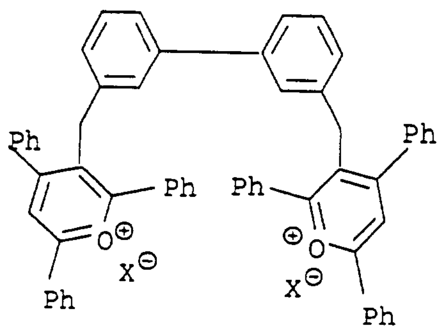

V,

where $X^{\ominus}$ is the equivalent of one anion, with phosphine in the temperature range from room temperature to 150°C in the presence of a catalytic amount of acid at elevated pressure in the presence or absence of a solvent in a pressure vessel.

**12.** A process as claimed in claim 11, wherein a phosphine partial pressure of from 0.5 to 50 bar is set in the reactor during the reaction.

**13.** A process as claimed in claim 11 or 12, wherein the phosphine is brought into contact with the pyrylium salt V at room temperature, this mixture is subsequently heated to above 100°C and after reaching this temperature the phosphine partial pressure in the pressure vessel is held essentially at from 0.5 to 50 bar during the reaction by passing in additional phosphine.

**14.** 2,4-Diphenyl-6-(2-naphthyl)phosphabenzene.

## Revendications

**1.** Procédé de préparation d'aldéhydes par hydroformylation d'oléfines avec $CO/H_2$, en présence de complexes de métaux de transition du sous-groupe VIII du Tableau Périodique des Eléments contenant des ligands sous forme de composés phosphorés, à des températures de 20 à 200°C et sous des pressions allant de la pression atmosphérique jusqu'à 700 bars, caractérisé en ce que le utilise en tant que ligands, des substances phospha-aromatiques ayant 5 à 100 atomes de C.

**2.** Procédé selon la revendication 1, caractérisé en que l'on utilise en tant que ligands, un phosphabenzène éventuellement substitué de formule I,

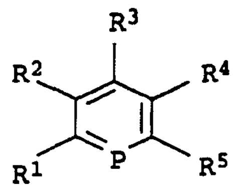

I,

dans laquelle $R^1$ à $R^5$ représente indépendamment l'un de l'autre, un atome d'hydrogène, un résidu alkyle, aralkyle ou aryle ou des résidus hétérocycliques ayant respectivement 1 à 12 atomes de C, encore les résidus - $COO^{\ominus}M^{\oplus}$, $-SO_3^{\ominus}M^{\oplus}$,

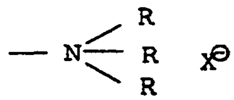

-OR, $-NR_2$, -COOR, -SR, $-(CHRCH_2O)_xR$ et $(CH_2CH_2NR)_x$, dans lesquels R représente des résidus identiques ou différents, choisis parmi un atome d'hydrogène ou les résidus aliphatiques ou aromatiques ayant 1 à 18 atomes de C, $M^{\oplus}$ est un cation et $X^{\ominus}$ un anion et x représente un nombre de 1 à 120 et dans lesquels les résidus $R^1$ à $R^5$ peuvent être liés à des cycles benzocondensés.

**3.** Procédé selon la revendication 2, caractérisé en ce que l'on utilise comme ligands, un phosphabenzène de formule I selon la revendication 2, qui comprend au moins un groupement phosphine ou phosphite dans au moins une chaîne latérale.

**4.** Procédé selon la revendication 1, caractérisé en ce que l'on utilise comme ligands, un composé bi ou polydenté de formule II

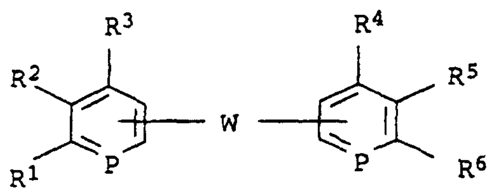

II,

dans laquelle $R^1$ à $R^6$ prennent les significations indiquées pour $R^1$ à $R^5$ dans la revendication 2 et W représente un pont sous forme d'une liaison covalente ou d'une chaîne de 1 à 10 atomes, qui peut être un élément constitutif d'un composé cyclique ou aromatique et où les substances phospha-aromatiques sont liées dans la position ortho ou méta par rapport à l'atome de phosphore, les positions ortho ou méta non utilisées pour le pont sont substituées par des résidus $R^1$ à $R^5$ selon la revendication 2, et où un ou plusieurs des substituants $R^1$ à $R^6$ peuvent représenter un phosphabenzène de formule I selon la revendication 2, lié par un pont W supplémentaire.

**5.** Phosphabenzènes bidentés de formule générale IIa

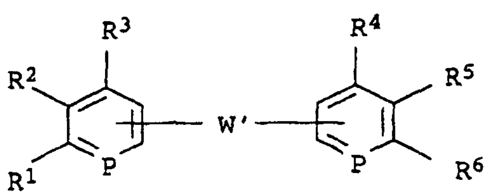

IIa,

dans laquelle les résidus $R^1$ à $R^6$ sont identiques ou différents et prennent les significations citées pour les résidus $R^1$ à $R^5$ dans la revendication 2 et dans laquelle W' représente un groupe 2,2'-, 4,4'- ou 3,3'-diphényldiyle, un groupe 2,2'-, 4,4'- ou 3,3'-diphényléther-diyle, un groupe 2,2'-, 4,4'- ou 3,3'-diphénylméthane-diyle ou un groupe

2,2'-, 4,4'- ou 3,3'-diphényléthane-diyle, un groupe 2,2'-, 4,4'- ou 3,3'-diphénylpropane-diyle ou un groupe ferrocène-diyle, qui est lié aux deux groupes phosphabenzènes dans la position ortho ou méta par rapport à leur atome de phosphore, à chaque fois par une liaison covalente, un groupe oxa et/ou un groupe alkyle en $C_1$ à $C_4$, en particulier un groupe méthylène, où le groupement diphényle, diphényléther ou diphényl-(alcane en $C_1$ à $C_3$) est non substitué ou substitué par 1 à 4 groupes alkyles en $C_1$ à $C_4$, identiques ou différents, et les positions ortho ou méta non utilisées pour le pont sont substituées par des résidus $R^1$ à $R^5$ selon la revendication 2, à l'exclusion des composés de formules VI

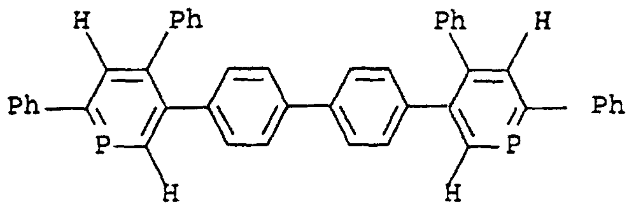

VI

et VII

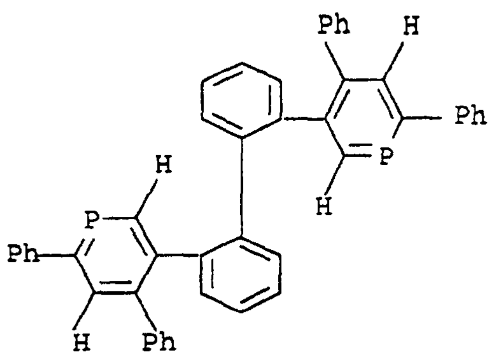

VII

dans lesquelles Ph représente un groupe phényle.

6. Phosphabenzène bidenté selon la revendication 5 de formule IV

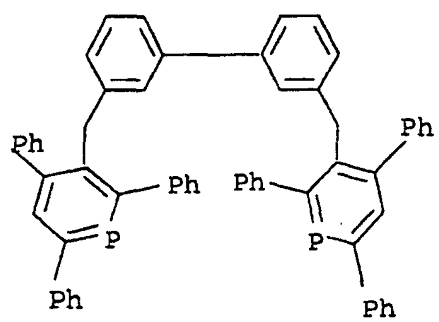

IV,

dans laquelle Ph représente un groupe phényle.

7. Procédé selon la revendication 1, caractérisé en ce que l'on utilise comme ligands, un phosphabenzène bidenté de formule générale IIa

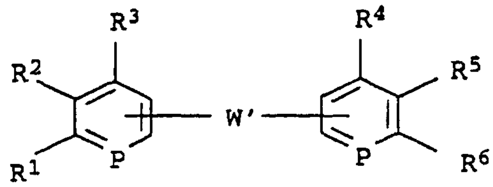

IIa,

dans laquelle $R^1$ à $R^6$ et W' prennent la signification citée dans la revendication 5.

**8.** Procédé selon la revendication 1, caractérisé en ce que l'on utilise comme ligands, le phosphabenzène bidenté de formule IV

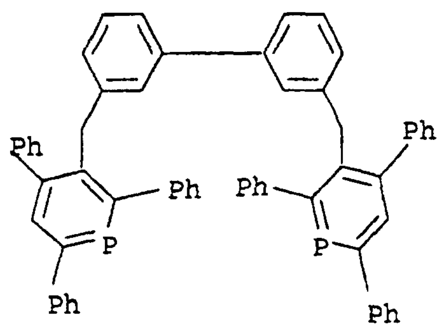

IV

.

**9.** Catalyseurs de formule III

$$M(L)_n(CO)_m \qquad III,$$

dans laquelle M représente au moins un atome central d'un élément du groupe secondaire VIII du Tableau Périodique des Eléments, L représente un ligand des formules I, II ou IIa ou IV

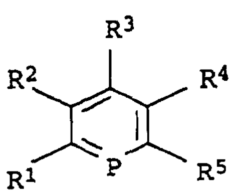

I

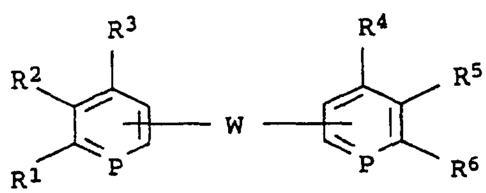

II,

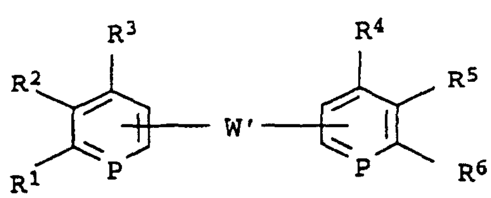

IIa,

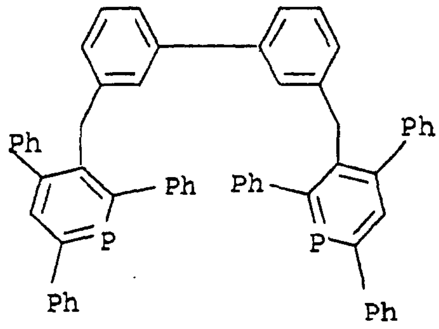

IV,

dans lesquelles $R^1$ à $R^6$, W et W' prennent les significations indiquées dans les revendications 2, 4 et 5, n et m valent chacun au moins 1 et où encore d'autres résidus, comme des résidus hydrure, alkyle ou acyle peuvent être contenus dans le complexe.

**10.** Catalyseurs selon la revendication 9, caractérisé en ce qu'ils sont préparés in situ dans des réactions d'hydroformylation ou de carbonylation.

**11.** Procédé de préparation du bis-3,3' (3(2,4,6-triphényl-phosphabenzène))-1,1'-diphényle de formule IV

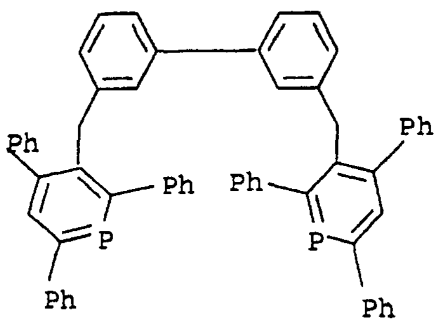

IV,

caractérisé en ce que l'on met le sel de pyrylium de formule V

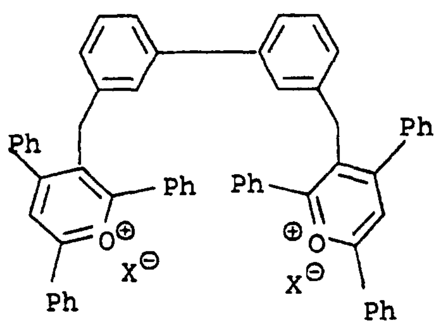

V,

dans laquelle $X^{\ominus}$ représente l'équivalent d'un anion, à réagir avec de l'hydrogène phosphoré dans la plage de températures allant de la température ambiante jusqu'à 150°C en présence d'une quantité catalytique d'acide sous une pression élevée en présence ou en absence d'un solvant, dans un récipient pressurisé.

**12.** Procédé selon la revendication 11, caractérisé en ce que l'on ajuste une pression partielle d'hydrogène phosphoré dans le réacteur de 0,5 à 50 bars pendant la réaction.

**13.** Procédé selon les revendications 11 et 12, caractérisé en ce que l'on amène l'hydrogène phosphoré à la température ambiante en contact avec le sel de pyrylium V, puis on chauffe ce mélange à une température supérieure à 100°C et après avoir atteint cette température, on maintient la pression partielle d'hydrogène phosphoré dans le récipient pressurisé pendant la réaction, essentiellement à un niveau de pression de 0,5 à 50 bars en introduisant de l'hydrogène phosphoré complémentaire.

**14.** Le 2, 4-diphényl-6(2-naphtyl)phosphabenzène.